# EUROPEAN PATENT SPECIFICATION

(11) **EP 1 768 710 B1**
(45) Date of publication and mention of the grant of the patent: **03.11.2010**
(21) Application number: 05773524.3
(22) Date of filing: 21.07.2005
(51) Int. Cl.: A61L 2/00, B32B 27/32, A61J 1/05

(54) **TRANSPARENT AUTOCLAVABLE BAG**
TRANSPARENTER AUTOKLAVIERBARER BEUTEL
SAC TRANSPARENT AUTOCLAVABLE

(30) Priority: 21.07.2004 US 896090
(43) Date of publication of application: 04.04.2007
(73) Proprietor: Amcor Flexibles Healthcare, Inc., Mundelein, IL 60060 (US)
(72) Inventor: INGRAHAM, Brian, Scott, Madison, WI 53707-7730 (US)
(74) Representative: Noel, Chantal Odile
(86) International application number: PCT/US2005/025986
(87) International publication number: WO 2006/012482

(56) References cited:
- EP-A- 1 013 193
- WO-A-93/06158
- US-A1- 2001 008 694
- US-A1- 2002 197 478
- US-A1- 2003 008 152

## Description

### FIELD OF THE INVENTION

The invention relates to a sterilization bag for medical applications, and to a composite film laminate suitable for producing such a bag.

### BACKGROUND OF THE INVENTION

Many healthcare products, including medical devices and pharmaceutical solutions, are stored until ready for use inside of sealed sterilization bags. Typically, the bags are in the form of a pouch, open along one side for receiving the medical instrument or other medical supply. The bag is then sealed and subjected to sterilization by exposure to gamma irradiation, electron beam, ultraviolet radiation, ethylene oxide, autoclaving, or other sterilization procedures.

In order to withstand the severe conditions of sterilization while providing high moisture barrier properties and long shelf life, sterilization bags this type have traditionally used composite laminate films with a barrier layer of either a metal foil or a metallized film. A disadvantage this type of laminate structure is that the bag is not transparent and therefore does not reveal the contents of the bag. To address this problem, some sterilization bags have provided a small window formed of a transparent barrier material. However, in order to maintain high barrier properties for the bag, the transparent window is kept relatively small in size. It would be desirable for a sterilization bag to have at least one transparent side to more clearly reveal the contents of the bag.

EP 1 013 193 A2 discloses a package for containing sample of a cosmetic.

This kind of package is not to be submitted to the severe condition of autoclaving.

WO 93/06158 discloses a high barrier oriented PCTFE film but does not disclose or suggest to use this barrier oriented PCTFE film in a laminate used to form an autoclavable bag.

US 2002/0197478 discloses a medical device container comprising_a multilayer material which can be made of a large variety of different layers.

### SUMMARY OF THE INVENTION

The present invention provides a transparent high barrier laminate material that can be suitably fabricated into sterilization bags, providing the ability to see the contents of the bag. The high barrier properties are achieved by using as the barrier layer a molecularly oriented polychlorotrifluoroethylene (PCTFE) film layer.

The autoclavable sterilization bag of the present invention comprises first and second composite film laminates positioned in opposing relation and sealed to one another to form a pouch. At least the first laminate comprises a heat sealable transparent thermoplastic polymer inner layer forming an inner surface of the laminate and a transparent polymer film outer layer. A transparent barrier layer is located between the inner and outer layers and formed of molecularly oriented polychlorotrifluoroethylene (PCTFE). In advantageous specific embodiments of the invention, the heat sealable transparent thermoplastic polymer inner layer is a polyolefin film, polyolefin copolymer, or coextrusions of either, and the transparent polymer outer layer is a film selected from the group consisting of polyethylene terephthalate, nylon, polypropylene, polyethylene and cellophane.

Polychlorotrifluoroethylene (PCTFE) fluoropolymer films are manufactured and sold by Honeywell Inc. under the trademark Aclar®. Non-oriented PCTFE films are used extensively by pharmaceutical companies in manufacturing transparent vacuum-formed blister packages for pharmaceuticals and for other healthcare packaging. However, PCTFE films have seen limited success in non-forming barrier applications in the flexible packaging industry.

The present invention is based upon the discovery that molecularly oriented PCTFE films provide a clear structure with sufficient barrier properties to replace metal foil in even the most demanding barrier applications utilizing ethylene oxide, gamma sterilization, e-beam sterilization, and autoclave sterilization techniques, as well as in non-sterilized applications. Molecularly orienting the PCTFE film decreases the moisture permeation rate significantly as compared to standard non-oriented PCTFE film and provides a very durable barrier layer with superior flex crack resistance.

For most polymer films, the process of molecularly orienting the film results in the film having poor dimensional stability at elevated temperature. When the film is reheated, the molecular chains tend to try to revert to their original non-oriented state, resulting in a dimensional change in the film. When the film is a component of a composite laminate, the dimensional change can result in warping, curling or other unsightly and undesirable changes in the product. Molecularly oriented PCTFE film has surprisingly good thermal dimensional stability, and thus is well suited for use in applications involving exposure to high temperatures, such as in an autoclave sterilization process.

The present invention also provides a dimensionally stable autoclavable composite film laminate that can be used in the fabrication of sterilization bags or other articles. The laminate comprises a heat sealable transparent thermoplastic polymer inner layer forming an inner surface of the laminate, a transparent polymer film outer layer, and a transparent barrier layer between the inner and outer layers and formed of molecularly oriented polychlorotrifluoroethylene (PCTFE). In advantageous embodiments of the invention, the heat sealable transparent thermoplastic polymer inner layer is a film or coating of a polyolefin, polyolefin copolymers, or coextrusions of either, and the transparent polymer outer layer is a film selected from the group consisting of polyethylene terephthalate, nylon, polypropylene, polyethylene and cellophane. The laminate may suitably include a layer of adhesive adhering the polyolefin film inner layer to the transparent barrier layer. The laminate may further include a layer of adhesive adhering the transparent polymer outer film layer to the barrier layer. In one specific embodiment, the dimensionally stable autoclavable composite film laminate comprises a heat sealable transparent polypropylene inner layer forming an inner surface of the laminate, a transparent biaxially oriented polyethylene terephthalate film outer layer, and a transparent barrier layer between the inner and outer layers and formed of molecularly oriented polychlorotrifluoroethylene (PCTFE).

### BRIEF DESCRIPTION OF THE DRAWINGS

Having thus described the invention in general terms, reference will now be made to the accompanying drawings, which are not necessarily drawn to scale, and wherein:
Figure 1 is a top plan view of a sterilization bag in accordance with one embodiment of the present invention.
Figure 2 is a cross-sectional view of a heat sealed edge portion of the bag taken substantially along the line 2-2 of Figure 1.
Figure 3 is a cross-sectional view showing one embodiment of a dimensionally stable composite film laminate useful in producing the sterilization bag of Figure 1.
Figure 4 is a cross-sectional view showing a second embodiment of a dimensionally stable composite film laminate in accordance with the present invention.

### DETAILED DESCRIPTION OF ILLUSTRATIVE EMBODIMENTS

The present inventions now will be described more fully hereinafter with reference to the accompanying drawings, in which some, but not all embodiments of the invention are shown. Indeed, these inventions may be embodied in many different forms and should not be construed as limited to the embodiments set forth herein; rather, these embodiments are provided so that this disclosure will satisfy applicable legal requirements. Like numbers refer to like elements throughout.

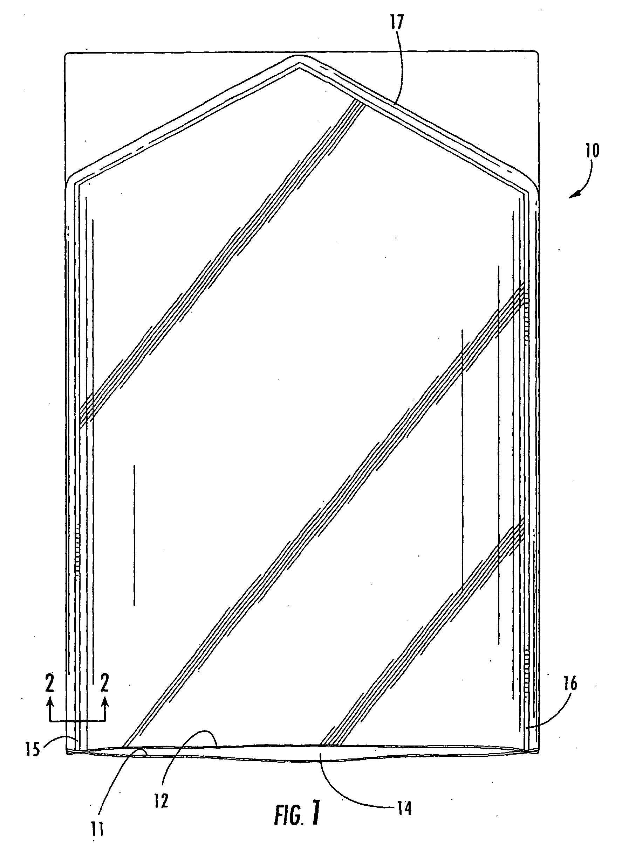

In Figure 1, the reference character 10 indicates an autoclavable sterilization bag in accordance with the present invention. The bag 10 is formed from first and second composite film laminates 11, 12 positioned in opposing face-to-face relation and sealed to one another along three sides to form a pouch-like structure, leaving an opening 14 along the remaining side suitable for receiving medical instruments or other articles for being sterilized. In the embodiment shown, the film laminates 11, 12 are generally rectangular in shape and they are sealed to one another along seal areas 15,16 adjacent opposite side edges of the bag, and along an angularly extending seal area 17 adjacent the top edge of the bag. After the bag 10 has been filled with its contents by the user, it may be sealed to close the opening 14. Sealing of the film laminates 11, 12 to one another may be carried out using conventional heat or ultrasonic sealing equipment as is well known in the art.

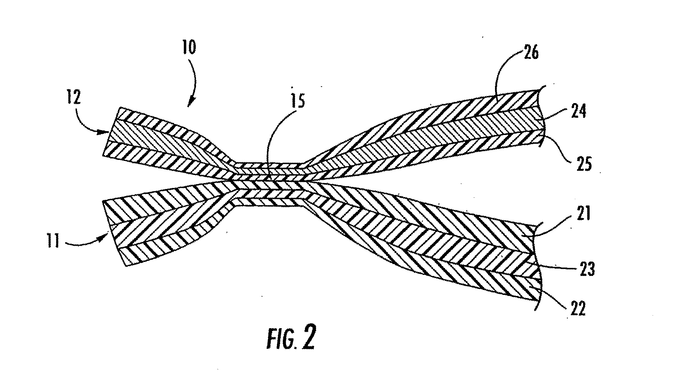

As shown in greater detail in Figure 2, the film laminate 11 includes three film layers, a heat sealable transparent thermoplastic polymer film inner layer 21 that forms an inner surface of the laminate, a transparent polymer film outer layer 22, and a transparent barrier film layer 23 between the inner and outer film layers 21, 22. The three film layers 21, 22, 23 are laminated to one another by adhesive. The thermoplastic polymer film inner layer 21 has an exposed surface that will soften and become adhesive upon exposure to heat or ultrasonic energy. The film layer 21 is preferably a polyolefin material, suitable examples of which include polypropylene, polyethylene, ethylene copolymers such as EAA, EMA, EVA and ionomer resins such as Surlyn® from DuPont or Iotek™ from ExxonMobil.. A particularly suitable film material for the inner film layer 21 is a cast polypropylene film made using a cast film extrusion process. The film layer 21 may suitably have a thickness of from about 0.5 mils to about 4.0 mils, more preferably about 1.5 to about 3.0 mils, and most preferably about 2 mils.

The transparent outer film layer 22 imparts strength, puncture resistance, dimensional stability and durability to the film laminate. The film layer 22 also assists in giving the laminate resistance to shrinkage when heated to elevated temperature. Suitable materials for the outer film 22 include polyethylene terephthalate (PET), nylon, polypropylene, polyethylene and cellophane. Particularly preferred are biaxially oriented films such as biaxially oriented PET and biaxially oriented nylon. The outer film layer 22 may have a thickness of from about 0.36 to 2.0 mils, more preferably from about 0.48 to 1.0 mils, and most preferably about 0.48 mils (48 ga).

The transparent barrier layer 23 imparts moisture barrier properties to the laminate. Barrier layer 23 is a molecularly oriented polychlorotrifluoroethylene (PCTFE) fluoropolymer film. The PCTFE film is transparent, biochemically inert, chemical resistant and free from plasticizers and stabilizers. Preferably the molecularly oriented PCTFE film is a monoaxially oriented film. The film may suitably have a thickness of from about 0.60 to 3 mils, more preferably from about 1 to 2 mils, and most preferably a thickness of about 1.5 mils. PCTFE fluoropolymer films are sold by Honeywell, Inc. under the Aclar® trademark.

The sealable inner film layer 21 may be laminated to the barrier layer 23 with an adhesive, using conventional lamination techniques. The adhesive may be applied using known processes such as spraying, roll coating, knife over roll coating, wire rod coating, or gravure coating. Suitable adhesives include solvent based, water based or solventless adhesives including acrylic adhesives, epoxy cured polyester urethanes, moisture cured polyester urethanes and isocyanate terminated polyester adhesives. Alternatively, the inner layer 21 can be formed directly on the barrier layer 23 by extrusion coating. The transparent outer layer 22 can be laminated directly to the barrier layer 23 using known adhesives and techniques as described above. If desired, the inner surface of the transparent outer layer 22 may be reverse printed prior to laminating to provide a layer of printing with graphics or other information. The outer layer may also be surface printed prior to or post lamination.

The film laminate 12 in the embodiment shown in Figure 2 includes a metal foil layer 24 with a sealable inner surface defined by a sealable film layer 25 of a thermoplastic polymer, such as a polyolefin. Alternatively, the sealable layer 25 can be a coating of a thermoplastic polymer such as a polyolefin. The foil layer 24 may comprise an aluminum foil having a thickness of from 0.275 mil to 1.50 mil. The sealable layer 25 may suitably have a thickness of about 0.5 mil to 4.0 mil. The foil layer 24 provides moisture barrier properties to the laminate. The coating layer 25 facilitates obtaining a strong seal with the heat sealable inner layer 21 of composite laminate 11. The laminate 12 additionally includes a protective outer film layer 26 formed of biaxially oriented PET laminated by an adhesive to the surface of the foil layer 24. The outer film layer 26 may be optionally reverse or surface printed with graphics or other information. In alternative embodiments, the metal layer may comprise a vacuum metallized moisture barrier layer deposited on a film layer such as PET.

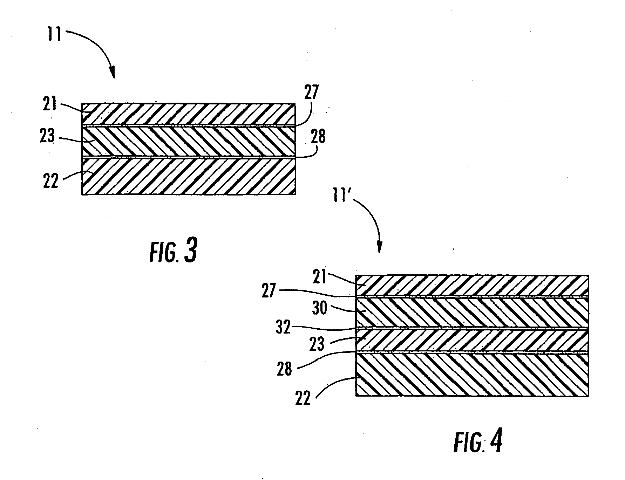

Figure 3 shows a cross-section of the composite film laminate 11 in greater detail. It will be seen that the inner layer 21 is laminated to the intermediate barrier layer 23 by an adhesive layer 27. The outer layer 22 is laminated to the opposite surface of the intermediate barrier layer 23 by an adhesive layer 28.
Figure 4 shows an alternative construction for a transparent composite film laminate 11' which is similar in many respects to the laminate 11 shown in Figures 2 and 3. To avoid repetitive description, corresponding reference numbers are used to identify corresponding elements wherever applicable. This embodiment differs over that of Figure 3 in that there is an additional intermediate film layer 30 located between the heat sealable inner layer 21 and the barrier layer 23. This intermediate barrier layer may suitably comprise a biaxially oriented nylon film or a biaxially oriented PET film, or biaxially oriented polypropylene. It may suitably have a thickness of from 0.36 to 2.0 mils. It is laminated to the inner film layer 21 by an adhesive layer 27 and to the intermediate barrier layer 23 by an adhesive layer 32.

In the embodiment shown and described, the autoclavable bag 10 has a transparent composite film laminate 11 on one side and an opaque metal layer-containing laminate 12 on the opposite side. The article contained within the bag is readily visible through the transparent film laminate 11. However, bags in accordance with the present invention can also be produced using the transparent composites film laminate 11 for both the front and back side of the bag.

### EXAMPLES

Example 1. A dimensionally stable composite film laminate was manufactured by first laminating a 0.48 mil biaxially oriented transparent polyethylene terephthalate (PET) film to a 1.5 mil thick monoaxially oriented transparent Aclar® polychlorotrifluoroethylene (PCTFE) film to form a two-layer composite. Rolls of the PET film and the Aclar® film were each mounted on unroll stands. The PET film was unrolled and directed across a gravure coating apparatus and a 1.5 lb/ream layer of urethane adhesive was applied to one surface. The Aclar® film was unrolled and then brought into contact with the adhesive-coated surface of the PET film, and the films were directed through a nip formed between two smooth surface rolls. A 2.0 mil polypropylene transparent film made using a cast film extrusion process was then laminated to the exposed surface of the Aclar® film by a similar laminating procedure. The resulting composite laminate exhibited high moisture barrier properties and excellent dimensional stability at elevated temperature. The 1.5 mil machine direction oriented Aclar® layer provided a moisture barrier of 0.0077 g/100 in²/24 hours at 100°F and 100% relative humidity.

Example 2. A laminating procedure similar to that described in Example 1 was used to produce the following metal laminate: 48 gauge biaxially oriented transparent PET film/ adhesive/ 35 gauge aluminum foil/ adhesive/ 2.0 mil cast polypropylene This tri-laminate was positioned opposite the laminate of Example 1, with the polypropylene surfaces facing one another and the two films were contacted with a heat sealing die to seal the two films together into a pouch of the configuration generally similar to that shown in Figure 1.

Example 3. A metal laminate of the following structure can be produced by a procedure similar to example 2: 0.48 mil biaxially oriented transparent PET film with a coating of aluminum deposited on one surface by vacuum metallization to an optical density of 2.8/ adhesive/ 2.0 mil cast polypropylene. A pouch is fabricated from this laminate and the laminate of Example 1.

Example 4. A pouch is produced by the procedure generally described Example 2, except that the following transparent laminate structure is substituted for the transparent laminate of example 1: 48 gauge biaxially oriented PET/adhesive/ 1.5 mil oriented Aclar® PCTFE film/adhesive/ 0.6 mil biaxially oriented nylon films/ adhesive/ 2.0 mil cast polypropylene.

Example 5. A pouch is produced by the procedure generally described example 2, except that the following transparent laminate structure is substituted for the transparent laminate of Example 1: 48 gauge biaxially oriented PET/adhesive/ 1.5 mil oriented Aclar® PCTFE film/adhesive/ 0.48 mil biaxially oriented PET film/ adhesive/ 2.0 mil cast polypropylene.

## Claims

1. An autoclavable sterilization bag (10) comprising first and second dimensionally stable composite film laminates (11, 11',12) positioned in opposing relation and sealed to one another to form a pouch, and wherein at least said first laminate (11, 11') comprises a heat sealable transparent thermoplastic polymer inner layer (21) forming an inner surface of the laminate, a transparent polymer film outer layer (22), and a transparent moisture barrier layer (23) between said inner and outer layers (21, 22) and formed of molecularly oriented polychlorotrifluoroethylene (PCTFE).

2. A bag according to claim 1, wherein said heat sealable transparent thermoplastic polymer inner layer (21) is a polyolefin film, and said transparent polymer outer layer (22) is a film selected from the group consisting of polyethylene terephthalate, nylon, polypropylene, polyethylene and cellophane.

3. A bag according to claim 1 or 2, including a layer (27) of adhesive adhering said polyolefin film inner layer to said transparent barrier layer.

4. A bag according to any one of the preceding claims , including a layer (20) of adhesive adhering said transparent polymer outer film layer (22) to said barrier layer (23).

5. A bag according to any one of the preceding claims, including an intermediate polymer film layer (30) between said polyolefin film inner layer (21) and said barrier layer (23), a layer (27) of adhesive between said polyolefin film inner layer and said intermediate polymer film layer, and a layer (32) of adhesive between said intermediate film layer and said barrier layer.

6. A bag according to claim 5, wherein said intermediate polymer film layer (30) is a biaxially oriented nylon film.

7. A bag according to claim 1 or 2, including a print layer reverse printed onto the interior surface of said transparent outer film layer (22).

8. A bag according to any one of the preceding claims, wherein said heat sealable transparent thermoplastic polymer inner layer (21) is a cast polypropylene film bonded directly to one surface of said barrier layer (23) by an adhesive, and said transparent polymer outer layer (22) is a biaxially oriented polyethylene terephthalate film bonded directly to an opposite surface of said barrier layer (23) by an adhesive.

9. A bag according to any one of the preceding claims, wherein said second composite film laminate (12) includes a metal layer (24).

10. A bag according to claim 9, wherein said second composite film laminate (12) includes a heat sealable polymer film inner layer (25) adhered to one surface of said metal layer (24) , and a biaxially oriented polymer film outer layer (26) adhered to an opposite surface of said metal layer (14).

11. A bag according to any one of the preceding claims, wherein said second composite film laminate (12) comprises a heat sealable transparent thermoplastic polymer inner layer (25) forming an inner surface of the laminate (12), a transparent polymer film outer layer (26), and a transparent moisture barrier layer (24) between said inner and outer layers (25, 26) and formed of molecularly oriented polychlorotrifluoroethylene (PCTFE).

12. A bag according to any one of the preceding claims , wherein said heat sealable transparent polymer inner layer (21) of said first laminate (11) comprises heat sealable transparent polypropylene forming said inner surface of the laminate, and said transparent polymer film outer layer (22) comprises a transparent biaxially oriented polyethylene terephthalate film.

13. A bag according to claim 12, including a first adhesive layer (27) located between and adhering together said polypropylene inner layer (21) and said transparent barrier layer (23), and a second adhesive layer (28) located between and adhering together said polyethylene terephthalate film outer layer (22) and said transparent barrier layer (23).

## Patentansprüche

1. Autoklavierbarer Sterilisationsbeutel (10), der erste und zweite dimensionsstabile Verbundfolien-Laminate (11, 11', 12) umfasst, die unter Bildung einer Tasche einander gegenüberliegend positioniert sind und aneinander gesiegelt sind, wobei wenigstens das erste Laminat (11, 11') eine heißsiegelbare transparente thermoplastische Polymer-Innenschicht (21), die die Innenseite des Laminats bildet, eine transparente Polymerfolien-Außenschicht (22) und eine transparente Feuchtigkeitsbarriereschicht (23) zwischen den Innen- und Außenschichten (21, 22) die aus molekular orientiertem Polychlortrifluorethylen (PCTFE) gebildet sind, umfasst.

2. Beutel nach Anspruch 1, wobei die heißsiegelbare transparente thermoplastische Polymer-Innenschicht (21) eine Polyolefinfolie ist und die transparente Polymer-Außenschicht (22) eine Folie ist, die aus der Gruppe, bestehend aus Polyethylenterephthalat, Nylon, Polypropylen, Polyethylen und Cellophan, ausgewählt ist.

3. Beutel nach Anspruch 1 oder 2, der eine Schicht (27) aus Klebstoff umfasst, der die Polyolefinfolien-Innenschicht an die transparente Barriereschicht klebt.

4. Beutel nach einem der vorangehenden Ansprüche, der eine Schicht (20) aus Klebstoff umfasst, die die transparente Polymer-Außenfolienschicht (22) an die Barriereschicht (23) klebt.

5. Beutel nach einem der vorangehenden Ansprüche, umfassend eine Polymerfolien-Zwischenschicht (30) zwischen der Polyolefinfolien-Innenschicht (21) und der Barriereschicht (23), eine Schicht (27) aus Klebstoff zwischen der Polyolefinfolien-Innenschicht und der Polymerfolien-Zwischenschicht und eine Schicht (32) aus Klebstoff zwischen der Folienzwischenschicht und der Barriereschicht.

6. Beutel nach Anspruch 5, wobei die Polymerfolien-Zwischenschicht (30) eine biaxial orientierte Nylonfolie ist.

7. Beutel nach Anspruch 1 oder 2, der eine Druckschicht umfasst, die spiegelbildlich auf die Innenseite der transparenten Folien-Außenschicht (22) gedruckt ist.

8. Beutel nach einem der vorangehenden Ansprüche, wobei die heißsiegelbare transparente thermoplastische Polymer-Innenschicht (21) eine gegossene Polypropylenfolie ist, die durch einen Klebstoff direkt an eine Oberfläche der Barriereschicht (23) geklebt ist, und die transparente Polymer-Außenschicht (22) eine biaxial orientierte Polyethylenterephthalat-Folie ist, die mit einem Klebstoff direkt an die gegenüberliegende Oberfläche der Barriereschicht (23) geklebt ist.

9. Beutel nach einem der vorangehenden Ansprüche, wobei das zweite Verbundfolien-Laminat (12) eine Metallschicht (24) umfasst.

10. Beutel nach Anspruch 9, wobei das zweite Verbundfolien-Laminat (12) eine heißsiegelbare Polymerfolien-Innenschicht (25), die an eine Oberfläche der Metallschicht (24) geklebt ist, und eine biaxial orientierte Polymerfolien-Außenschicht (26), die an eine gegenüberliegende Oberfläche der Metallschicht (14) geklebt ist, umfasst.

11. Beutel nach einem der vorangehenden Ansprüche, wobei das zweite Verbundfolien-Laminat (12) eine heißsiegelbare transparente thermoplastische Polymer-Innenschicht (25), die eine Innenseite des Laminats (12) bildet, eine transparente Polymerfolien-Außenschicht (26) und eine transparente Feuchtigkeitsbarriereschicht (24) zwischen den Innen- und Außenschichten (25, 26), die aus molekular orientiertem Polychlortrifluorethylen (PCTFE) gebildet sind, umfasst.

12. Beutel nach einem der vorangehenden Ansprüche, wobei die heißsiegelbare transparente Polymer-Innenschicht (21) des ersten Laminats (11) heißsiegelbares transparentes Polypropylen, das die Innenseite des Laminats bildet, umfasst, und die transparente Polymerfilm-Außenschicht (22) eine transparente biaxial orientierte Polyethylenterephthalat-Folie umfasst.

13. Beutel nach Anspruch 12, umfassend eine erste Klebstoffschicht (27), die zwischen der Polypropylen-Innenschicht (21) und der transparenten Barriereschicht (23) angeordnet ist und diese miteinander verklebt, und eine zweite Klebstoffschicht (28), die zwischen der Polyethylenterephthalat-Folien-Außenschicht (22) und der transparenten Barriereschicht (23) angeordnet ist und diese miteinander verklebt.

## Revendications

1. Sac de stérilisation autoclavable (10) comprenant un premier et un second stratifiés de films composites aux dimensions stables (11, 11', 12) positionnés de manière opposée et scellés l'un à l'autre afin de former une poche, et dans lequel au moins ledit premier stratifié (11, 11') comprend une couche interne de polymère thermoplastique transparent pouvant être scellé à chaud (21) formant une surface interne du stratifié, une couche externe de film polymère transparent (22), et une couche transparente barrière d'humidité (23) entre lesdites couches interne et externe (21, 22) et formée de polychlorotrifluoroéthylène (PCTFE) moléculairement orienté.

2. Sac selon la revendication 1, dans lequel ladite couche interne de polymère thermoplastique transparent pouvant être scellé à chaud (21) est un film de polyoléfine, et ladite couche externe de polymère transparent (22) est un film choisi dans le groupe comprenant le polyéthylène téréphtalate, le nylon, le polypropylène, le polyéthylène et la cellophane.

3. Sac selon la revendication 1 ou 2, comprenant une couche (27) d'adhésif faisant adhérer ladite couche interne de film polyoléfinique à ladite couche transparente barrière.

4. Sac selon l'une quelconque des revendications précédentes, comprenant une couche (20) d'adhésif faisant adhérer ladite couche externe de film polymère transparent (22) à ladite couche barrière (23).

5. Sac selon l'une quelconque des revendications précédentes, comprenant une couche intermédiaire de film polymère (30) entre ladite couche interne de film polyoléfinique (21) et ladite couche barrière (23), une couche (27) d'adhésif entre ladite couche interne de film polyoléfinique et ladite couche intermédiaire de film polymère, et une couche (32) d'adhésif entre ladite couche intermédiaire de film et ladite couche barrière.

6. Sac selon la revendication 5, dans lequel ladite couche intermédiaire de film polymère (30) est un film en nylon orienté de manière biaxiale.

7. Sac selon la revendication 1 ou 2, comprenant une couche d'impression imprimée en inverse sur la surface intérieure de ladite couche externe de film transparent (22).

8. Sac selon l'une quelconque des revendications précédentes, dans lequel ladite couche interne de polymère thermoplastique transparent pouvant être scellé à chaud (21) est un film en polypropylène moulé lié directement à une surface de ladite couche barrière (23) par un adhésif, et ladite couche externe de polymère transparent (22) est un film en polyéthylène téréphtalate orienté de manière biaxiale lié directement à une surface opposée de ladite couche barrière (23) par un adhésif.

9. Sac selon l'une quelconque des revendications précédentes, dans lequel ledit second stratifié de film composite (12) comprend une couche métallique (24).

10. Sac selon la revendication 9, dans lequel ledit second stratifié de film composite (12) comprend une couche interne de film polymère pouvant être scellé à chaud (25) adhéré à une surface de ladite couche métallique (24), et une couche externe de film polymère orienté de manière biaxiale (26) adhéré à une surface opposée de ladite couche métallique (14).

11. Sac selon l'une quelconque des revendications précédentes, dans lequel ledit second stratifié de film composite (12) comprend une couche interne de polymère thermoplastique transparent pouvant être scellé à chaud (25) formant une surface interne du stratifié (12), une couche externe de film polymère transparent (26), et une couche transparente barrière d'humidité (24) entre lesdites couches interne et externe (25, 26) et formée de polychlorotrifluoroéthylène (PCTFE) moléculairement orienté.

12. Sac selon l'une quelconque des revendications précédentes, dans lequel ladite couche interne de polymère transparent pouvant être scellé à chaud (21) dudit premier stratifié (11) comprend un polypropylène transparent pouvant être scellé à chaud formant ladite surface interne du stratifié, et ladite couche externe de film polymère transparent (22) comprend un film de polyéthylène téréphtalate transparent orienté de manière biaxiale.

13. Sac selon la revendication 12, comprenant une première couche adhésive (27) localisée entre et faisant adhérer ensemble ladite couche interne de polypropylène (21) et ladite couche transparente barrière (23), et une seconde couche adhésive (28) localisée entre et faisant adhérer ensemble ladite couche externe de film de polyéthylène téréphtalate (22) et ladite couche transparente barrière (23).
